# EUROPÄISCHE PATENTSCHRIFT

(11) **EP 2 655 319 B1**
(45) Veröffentlichungstag und Bekanntmachung des Hinweises auf die Patenterteilung: **08.05.2019**
(21) Anmeldenummer: 11807900.3
(22) Anmeldetag: 19.12.2011
(51) Int. Cl.: C07C 253/30, C07C 255/46

(54) **VERFAHREN ZUR HERSTELLUNG VON CIS-1-AMMONIUM-4-ALKOXYCYCLOHEXANCARBONITRILSALZEN**
METHOD FOR PRODUCING CIS-1-AMMONIUM-4-ALKOXYCYCLOHEXANECARBONITRILE SALTS
PROCÉDÉ DE PRODUCTION DE SELS DE CIS-1-AMMONIUM-4-ALKOXYCYCLOHEXANCARBONITRILE

(30) Priorität: 22.12.2010 EP 10196473; 29.12.2010 US 201061427913 P
(43) Veröffentlichungstag der Anmeldung: 30.10.2013
(73) Patentinhaber: Bayer CropScience Aktiengesellschaft, 40789 Monheim (DE)
(72) Erfinder: VOLZ, Frank, 50825 Köln (DE); SCHNATTERER, Albert, 51373 Leverkusen (DE); KAPTEIN, Bernardus, NL-6133 BE Sittard (NL); CASTELIJNS, Anna, Maria, Cornelia, Francisca, NL-6176 JB Spaubeek (NL)
(74) Vertreter: BIP Patents
(86) Internationale Anmeldenummer: PCT/EP2011/073292
(87) Internationale Veröffentlichungsnummer: WO 2012/084862

(56) Entgegenhaltungen:
- WO-A1-2008/128058
- L. MUNDAY: "Alkylcyclohexanones in the Strecker and Bucherer Hydantoin syntheses", NATURE, Bd. 190, 17. Juni 1961 (1961-06-17), Seiten 1103-1104, XP002637675, in der Anmeldung erwähnt

## Beschreibung

Die vorliegende Erfindung betrifft ein neues Verfahren zur Herstellung von *cis*-1-Ammonium-4-alkoxycyclohexancarbonitrilsalzen sowie neue Intermediate bzw. Ausgangsverbindungen, welche in dem erfindungsgemäßen Verfahren durchlaufen bzw. verwendet werden. *Cis*-1-Ammonium-4-alkoxycyclohexancarbonitrilsalze sind wichtige Zwischenprodukte zur Synthese von insektiziden Wirkstoffen.

Substituierte cyclische Ammoniumsalze sind durch Umsetzung der entsprechenden Aminonitrile mit Säuren zugänglich. Dabei fallen Verbindungen der Formel (I) 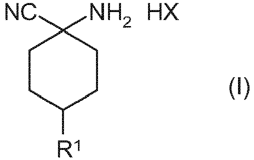 als Isomerengemisch der Formeln (cis-I) und (trans-I) 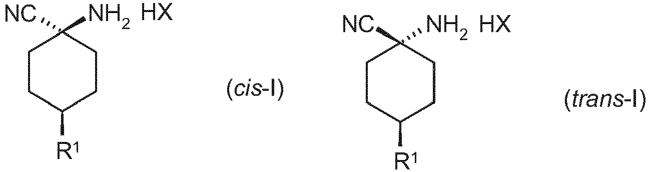 an.

Salze entsprechender 4-substituierter 1-Amino-cyclohexancarbonitrile sind in der Literatur beschrieben. (L. Munday, J. Chem. Soc. 1961, 4372-4379; L. Munday, Nature 1961, 190, 1103-1104; Y. Maki, T. Masugi, Chem. Pharm. Bull. 1973, 21, 685-691; J. T. Edward, C. Jitrangsri, Can. J. Chem. 1975, 53, 3339-3350). Bei den hier beschriebenen Salzen handelt es sich ausschließlich um Hydrochloride der entsprechenden 1-Amino-cyclohexancarbonitrile. Diese wurden entweder mit einem ungünstigen cis/trans-Isomerenverhältnis bis hin zur reinen trans-Verbindung isoliert. Dies ist nicht verwunderlich, da für Strecker Reaktionsprodukte (Aminonitrile) und folglich deren Salze, eine entsprechende Stereochemie in der oben genannten Literatur beschrieben ist. Da für den hier beschriebenen Prozess das cis-Isomere gewünscht ist, sind die literaturbekannten Verfahren nicht geeignet.

Die Verbindungen der Formeln (*cis*-I) und (*trans-*I) sind nach dem in EP-A-0595130 und WO 2003/080557 beschriebenen Verfahren herstellbar, die Verbindungen der Formeln (*cis*-I) und (trans-I) sind neu.

Die Synthese des *cis*/*trans*-Isomerengemisches als entsprechendes Hydrochlorid wurde als Zwischenverbindung bereits in WO 2008/128058 beschrieben. Auf eine Trennung der beiden Isomere wurde allerdings verzichtet und damit ist die selektive Herstellung der cis-Verbindung nicht beschrieben.

Die Aufgabe der vorliegenden Erfindung besteht in der Bereitstellung eines neuen, wirtschaftlichen Verfahrens zur selektiven Herstellung von *cis*-1-Ammonium-4-alkoxycyclohexancarbonitrilsalzen ausgehend von cis/trans-Amino-4-alkoxycyclohexancarbontrilen.

Gegenstand der vorliegenden Erfindung ist ein Verfahren zur Herstellung von Verbindungen der Formeln (*cis*-I) und (*trans*-I) 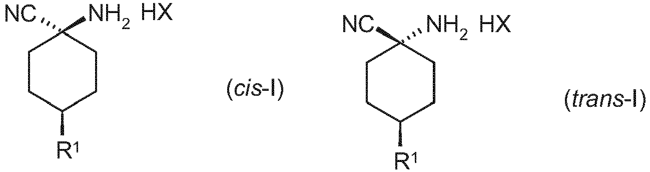 in welcher
- R¹: für OR²,
- R²: für Alkyl,
- HX: für HCl, H₂SO₄, H₃PO₄, HCOOH, HO₂CCO₂H, p-Toluolsulfonsäure oder Methansulfonsäure steht.

Die Strecker Reaktion wird im allgemeinen derart durchgeführt, dass man ein substituiertes cyclisches Keton der Formel (II) 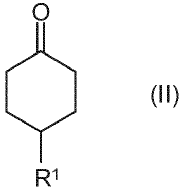 in welcher R¹ die oben angegebenen Bedeutungen hat
mit Alkalicyanid, Ammoniumchlorid und Ammoniak (oder direkt mit Blausäure und Ammoniak) in Gemischen von organischen Lösungsmitteln und Wasser im Zwei-Phasen-System umsetzt und das dabei entstehende Aminonitril der Formel (III) 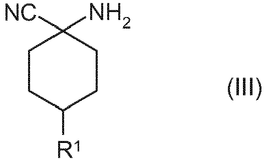 in welcher R¹ die oben angegebenen Bedeutungen hat
isoliert.

Dabei fallen die Aminonitrile der Formel (III) üblicherweise als Gemische aus
*cis*-Isomer (cis-III) 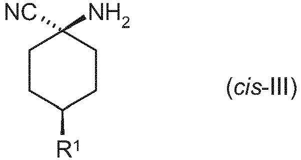 und *trans*-Isomer (trans-III) 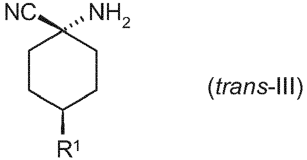 an.

Diese substituierten cyclischen Aminonitrile der Formel (III) lassen sich durch Umsetzung mit Säuren (HX) in organischen Lösungsmitteln zu Gemischen der entsprechenden Ammoniumsalze der Formel (I) 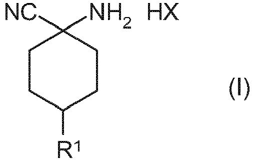 überführen.

Dabei fallen die Verbindungen der Formel (I) als Gemische aus cis-Isomer (*cis*-I) und trans-Isomer (*trans*-I) an.

Es wurden neue Verbindungen der Formeln (*cis*-I und (*trans*-I) gefunden 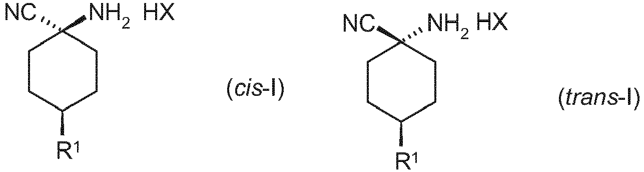 in welchen
R¹ und HX die oben angegebenen Bedeutungen haben.

Bevorzugt sind Verbindungen der Formeln (*cis*-I) und (*trans*-I), in welchen
- R¹: für OR²,
- R²: für Alkyl,
- HX: für H₂SO₄, H₃PO₄, HCOOH, HO₂CCO₂H, p-Toluolsulfonsäure oder Methansulfonsäure steht.

Besonders bevorzugt sind Verbindungen der Formeln (*cis*-I) und (*trans*-I), in welchen
- R¹: für OR²,
- R²: für C₁-C₆-Alkyl,
- HX: für H₂SO₄, H₃PO₄, HCOOH oder HO₂CCO₂H steht.

Ganz besonders bevorzugt sind Verbindungen der Formeln (*cis*-I) und (*trans*-I), in welchen
- R¹: für OR²,
- R²: für Methyl, Ethyl oder n-Propyl, (hervorgehoben für Methyl),
- HX: für HCOOH steht.

Für manche Verbindungen (beispielsweise aus EP-A- 596 298, WO 95/26954, WO95/20572, EP-A 668 267, WO 96/25395, WO 96/35664, WO 97/01535, WO 97/02243, WO 97/36868, WO 98/05638, WO 99/43649, WO 99/48869, WO 99/55673, WO 01/23354, WO 01/74770) werden substituierte cyclische Ammoniumnitrilsalze der allgemeinen Formel (I) als Zwischenprodukte benötigt.

Dabei kann es für bestimmte dieser beispielsweise aus EP-A- 596 298, WO 95/26954, WO95/20572, EP-A 668 267, WO 96/25395, WO 96/35664, WO 97/01535, WO 97/02243, WO 97/36868, WO 98/05638, WO 99/43649, WO 99/48869, WO 99/55673, WO 01/23354, WO 01/74770 bekannt gewordenen Verbindungen von Vorteil sein, das entsprechende cis-konfigurierte Ammoniumnitrilsalz der Formel (*cis*-I) einzusetzen.

Überraschenderweise lassen sich nach dem erfindungsgemäßen Verfahren das *cis*/*trans-*Isomerengemisch an Aminonitrilen (*cis*-III und *trans-*III) über ihre entsprechenden Ammoniumsalze der Formel (cis-I und trans-I) aufgrund unterschiedlicher Löslichkeit in organischen Lösungsmitteln, auftrennen.

Als Alkalicyanide für die Synthese des *cis*/*trans*-Isomerengemisches der Formel (III) können bevorzugt Lithiumcyanid, Natriumcyanid oder Kaliumcyanid verwendet werden, besonders bevorzugt sind Natrium- oder Kaliumcyanid, ganz besonders bevorzugt ist Natriumcyanid.

Die Menge an Alkalicyanid bezogen auf das Keton der Formel (II) liegt zwischen 0.8 und 3 Mol. Bevorzugt werden Mengen zwischen 1 und 2.5 Mol bezogen auf das Keton der Formel (II) eingesetzt; besonders bevorzugt sind Mengen zwischen 1 und 1.5 Mol pro Mol Keton (II).

Die Menge an Ammoniumchlorid beträgt zwischen 0.9 und 3.5 Mol pro Mol Keton (II). Bevorzugt werden Mengen zwischen 1 und 3 Mol pro Mol Keton (II); besonders bevorzugt sind Mengen zwischen 1.1 und 2 Mol pro Mol Keton (II).

Die Menge an Ammoniak bezogen auf das Keton der Formel (II) liegt zwischen 1 und 8 Mol. Bevorzugt werden Mengen zwischen 1 und 5 Mol bezogen auf das Keton der Formel (II) eingesetzt; besonders bevorzugt sind Mengen zwischen 1 und 3 Mol pro Mol Keton (II).

Die Reaktionstemperatur des erfindungsgemäßen Verfahrens liegt zwischen 20 und 100°C; bevorzugt ist ein Temperaturbereich von 20 bis 70°C.

Geeignete Lösungsmittel sind bevorzugt Wasser, sowie Alkohol (z. Bsp. Methanol, Ethanol, Propanol, Butanol) /Wasser-Gemische, sowie Zweiphasensysteme bestehend aus Wasser und organischen Lösungsmitteln wie Methyl-tert.-butylether, Toluol, Essigsäurealkylester (Alkyl = z. Bsp. Methyl Ethyl,), Tetrahydrofuran. Besonders bevorzugte Lösungsmittel sind Wasser, Wasser/Alkohol Gemische und folgende Zweiphasensysteme bestehend aus Wasser und organischen Lösungsmitteln wie MTBE, Toluol, Essigsäureethylester oder Tetrahydrofuran.

Die Isolierung des Isomerengemisches an *cis*/*trans*-Aminonitril der Formel (III) erfolgt durch Flüssig/Flüssig-Extraktion. Die erhaltenen Aminonitrile der Formel (III) können mit organischen Lösungsmitteln, wie Toluol, Essigsäurealkylester (Alkyl = z. Bsp. Methyl Ethyl,), Methyl-tert.-butylether, Toluol Tetrahydrofuran extrahiert werden.

Zur erhaltenen Lösung des Isomerengemisches an *cis*/*trans-*Aminonitrile (III) in einem der oben genannten organischen Lösungsmitteln, bevorzugt in Methyl-tert.-butylether, Toluol, Essigsäurealkylester (Alkyl = z. Bsp. Methyl Ethyl,), Tetrahydrofuran und besonders bevorzugt in Methyl-tert.-butylether, Toluol, Essigsäureethylester, Essigsäuremethylester, Tetrahydrofuran wird Säure zugegeben. Bevorzugt sind Salzsäure, Schwefelsäure, Phosphorsäure, Ameisensäure, Oxalsäure, p-Toluolsulfonsäure oder Methansulfonsäure, besonders bevorzugt sind Schwefelsäure, Phosphorsäure, Ameisensäure, Oxalsäure, p-Toluolsulfonsäure oder Methansulfonsäure, ganz besonders bevorzugt sind Ameisensäure, Schwefelsäure, Oxalsäure oder Phosphorsäure, hervorgehoben ist Ameisensäure. Die Isolierung der erhaltenen Feststoffe der Formel (I) erfolgt durch Filtration des Reaktionsgemisches und Trocknen des Filterrückstandes.

Das erfindungsgemäße Verfahren kann beispielhaft durch folgendes Schema veranschaulicht werden:

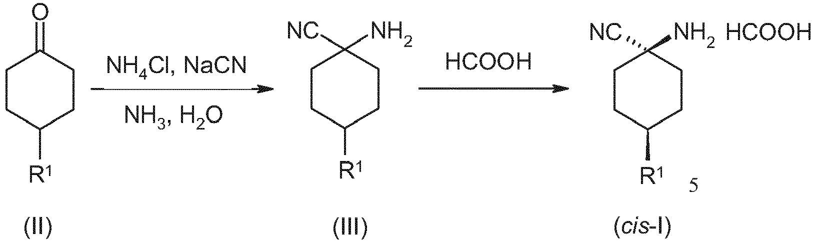

Ebenfalls Gegenstand dieser Erfindung ist ein Verfahren zur Isomerisierung des unerwünschten trans-Isomeren der Formel (*trans-*III)*,* 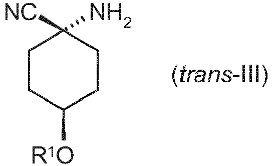 in welcher
R¹ die oben genannten Bedeutungen hat
bzw. das Ammoniumsalz der Formel (*trans*-I), 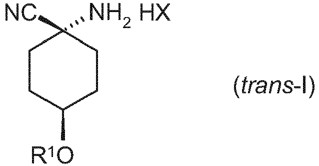 in welcher
R¹, R² und HX die oben angegebenen Bedeutungen haben
zu einem *cis*/*trans*-Isomerengemisch der Formel (III), wie es bei *Strecker*-Reaktion anfällt.

Das erfindungsgemäße Verfahren kann beispielhaft durch folgendes Schema verdeutlicht werden: 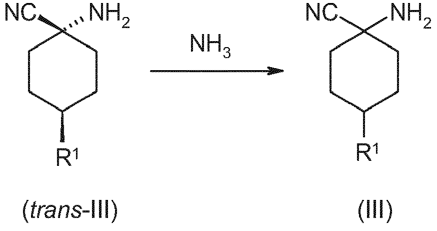 bzw.

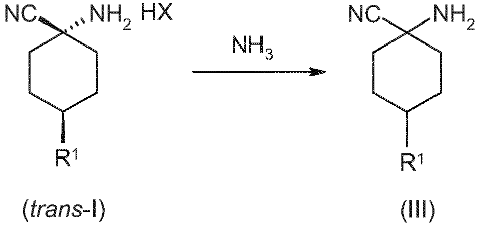

Die Menge an Ammoniak bezogen auf das Keton der Formel (II) liegt zwischen 1 und 5 Mol. Bevorzugt werden Mengen zwischen 1 und 3 Mol bezogen auf das Keton der Formel (III) eingesetzt; besonders bevorzugt sind Mengen zwischen 1 und 2 Mol pro Mol Keton (III).

Als Lösungsmittel wird ein Gemisch aus Wasser/Ammoniak oder entsprechend die oben beschriebenen Lösungsmittel verwendet.

Die Reaktionstemperatur des erfindungsgemäßen Verfahrens liegt zwischen 20 und 100°C; bevorzugt ist ein Temperaturbereich von 20 bis 60°C, besonders bevorzugt 50 - 60°C.

Überraschenderweise kann nach dem erfindungsgemäßen Verfahren die Isomerisierung der unerwünschten *trans*-Isomere (*trans-*I und *trans-*III) unter den Bedingungen der *Strecker*-Reaktion durchgeführt werden, was aus verfahrenstechnischer Sicht vorteilhaft ist. Darüber hinaus ist es möglich die *Strecker*-Reaktion in einem 2-Phasen-Gemisch aus organischen Lösungsmitteln und Wasser durchzuführen, was aus verfahrenstechnischer Sicht sehr effizient ist. Derartige Beispiele für diese Vorgehensweise sind in der Literatur nicht beschrieben.

Das erfindungsgemäße Verfahren kann beispielhaft durch folgendes Schema verdeutlicht werden:

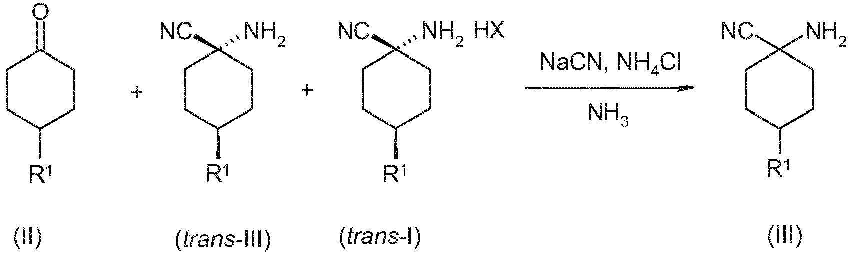

Die Durchführung erfolgt analog zur *Strecker*-Reaktion*.* Zur vollständigen Isomerisierung der beigemischten *trans*-Isomere (*trans*-III und *trans*-I) muss die Ammoniakmenge um 2-5 Mol pro Mol *trans*-Isomere (*trans-*III und *trans-*I)*,* bevorzugt 2-3 Mol und besonders bevorzugt um 1.5-2 Mol erhöht werden.

Die Reaktionstemperatur des erfindungsgemäßen Verfahrens liegt zwischen 20 und 80°C, bevorzugt ist ein Temperaturbereich von 30-70°C.

### Herstellungsbeispiele:

### Beispiel 1

### Cis/trans-1-Amino-4-methoxycyclohexancarbonitril

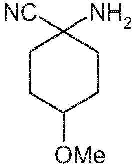

Zu einer Lösung von 4-Methoxycyclohexanon (204.4 g, 97.9%ig (laut GC), 1.56 mol, frisch destilliert: 59.9 °C/ 3 mbar), NH₄Cl (92.3 g, 1.1 Äq., 1.72 mol, 99.5%ig, als gesättigte Lösung in H₂O (250g)), Toluol (475 g, 30 Gew.-% vom gesamten Reaktionsansatz) wird unter starkem Rühren (KPG-Rührer) NaCN (79.6 g, 1 Äq., 1.56 mol, 96%ig, als gesättigte Lösung in H₂O und Ammoniak (265.3 g, 2.5 Äq., 3.9 mol, 25%ig in H₂O) innerhalb von 1.5 Stunden zugetropft. Nach 1 Stunde Nachrührzeit werden die Phasen getrennt und die wässrige Phase mit Toluol extrahiert. Die toluolische Phase wird unter reduziertem Druck auf eine Gesamtmenge von 700 g destilliert (entfernt man die gesamte Menge an Toluol so bleiben 246.2 g Öl mit einem Gehalt (Fl-% GC) von durchschnittlich 85%. Daraus resultieren: 209.3g *cis*/*trans*-1-Amino-4-methoxycyclohexancarbonitril (1.36 mol, 87%, *cis : trans* = 55 : 45, GC-MS: [M]⁺: m/z = 154, [M-H]⁺: m/z = 153).

¹H-NMR (600 MHz, (CD₃)₂SO): δ = 9.63 (br. S, 2H, NH₂), 3.46 (m, 1H, CHOMe, *cis*), 3.25 (s, 3H, Me, *trans*), 3.22 (s, 3H, Me, *cis*)*,* 3.15 (m, 1H, CHOMe, *trans*), 1.29 - 2.27 (m, 8H, CH₂).
¹³C-NMR (150 MHz, (CD₃)₂SO): δ = 117.94 (CN, *cis* + *trans*), 75.7 (CHOMe, *trans*), 71.2 (CHOMe, *cis*), 55.6 (H₃CO, *trans*), 55.4 (H₃CO, *cis*), 51.5 (CNH₂, *cis*), 51.3 (CNH₂, *trans*), 31.4 (H₂NCCH₂, *trans*), 28.1(H₂NCCH₂, *cis*), 27.6(MeOCHCH₂, *trans*), 25.6(MeOCHCH₂, *cis*)*.*
GC-MS:[M]⁺: m/z = 154.

### Beispiel 2

### Synthese von cis-1-Amino-4-methoxycyclohexancarbonitril-hydroformiat

Zur in Beispiel 1 erhaltenen toluolischen Lösung an *cis*/*trans*-1-Amino-4-methoxycyclohexancarbonitril werden 1.4 kg MTBE hinzugefügt. Unter Rühren werden bei Raumtemperatur 1 Mol-% Impfkristalle *cis*-1-Amino-4-methoxycyclohexancarbonitril-hydroformiat (2.73 g, 13.6 mmol) hinzugefügt. Dann wird Ameisensäure (32.3 g, 26.5 mL, 0.7 mol) innerhalb von 15 Minuten hinzugetropft und die erhaltene Lösung mit einem Eisbad für 2 Stunden gekühlt. Das ausgefallene Ammoniumformiat wird abfiltriert und der Filterkuchen mit Toluol und MTBE gewaschen. Nach Trocknung werden *cis*-1-Amino-4-methoxycyclohexancarbonitril-hydroformiat (118.7 g, Gehalt: GC-Fl.-% nach Derivatisierung mit Trifluoressigsäureanhydrid: 82.8 %, cis/trans = 95 : 5, daraus resultieren: 98.3 g, 0.49 mol, 32% (bez. auf 4-Methoxycyclohexanon), LC-MS: [M-O₂CH⁺]: m/z = 155.1) als farblosen Feststoff erhalten.

### Beispiel 3

### Isomerisierung und Strecker-Reaktion

Die Mutterlauge wird für die Rückführung wie folgt vorbereitet: Zuerst wird MTBE unter reduziertem Druck weitestgehend entfernt und die toluolische Lösung in einen 2 L Dreihalskolben überführt. Dann wird 4-Methoxycyclohexanon (62.8 g, 97.9%ig, 0.49 mol) abgetrenntem *cis*-1-Amino-4-methoxycyclohexancarbonitril-hydroformiat aus Beispiel 2), NH₄Cl (29.0 g, 1.1 Äq., 0.49 mol, 99.5%ig, als gesättigte Lösung in H₂O) und Toluol (475 g) zugefügt. Unter starkem Rühren wird NaCN (25.0 g, 1 Äq., 0.49 mol, 96%ig, als gesättigte Lösung in H₂O) und Ammoniak (83.3 g, 2.5 Äq., 1.22 mol, 25%ig in H₂O, zusätzlich für die Isomerisierung des rückgeführten Aminonitrils: NH₃ (47.9 g, 0.702 mol, 25%ig in H₂O)) separat über 1.5 Stunden zudosiert. Nach 1 Stunde bei 60°C wurden die Phasen getrennt und die wässrige Phase mit Toluol extrahiert. Nach Entfernen des Toluols unter reduziertem Druck werden 242.3 g Öl mit einem Gehalt (Fl-% GC) von durchschnittlich 85% erhalten. Daraus resultieren: 206.0 g *cis*/*trans*-1-Amino-4-methoxycyclohexancarbonitril (1.34 mol, 85%, *cis* : *trans* = 55 : 45, GC-MS: [M]⁺: m/z = 154, [M-H]⁺: m/z = 153).

## Patentansprüche

1. Verbindungen der Formeln (*cis-*I) und (*trans*-I) 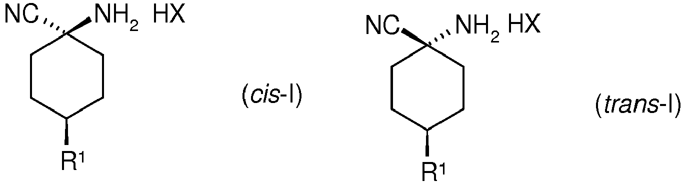 in welcher
R¹ für OR²,
R² für Alkyl,
HX für HCl, H₂SO₄, H₃PO₄, HCOOH, HO₂CCO₂H, p-Toluolsulfonsäure oder Methansulfonsäure steht.

2. Verbindungen der Formeln (*cis-*I) und (trans-I) gemäß Anspruch 1, in welcher
R¹ für OR²,
R² für Alkyl,
HX für H₂SO₄, H₃PO₄, HCOOH, HO₂CCO₂H, p-Toluolsulfonsäure oder Methansulfonsäure steht.

3. Verbindungen der Formeln (*cis-*I) und (trans-I) gemäß Anspruch 1, in welcher
R¹ für OR²,
R² für C₁-C₆-Alkyl,
HX für H₂SO₄, H₃PO₄, HCOOH oder HO₂CCO₂H steht.

4. Verbindungen der Formeln (*cis-*I) und (*trans*-I) gemäß Anspruch 1, in welcher
R¹ für OR²,
R² für Methyl, Ethyl oder n-Propyl,
HX für HCOOH steht.

5. Verbindungen der Formel (cis-I) gemäß Anspruch 1, in welcher R¹ und HX die oben angegebenen Bedeutungen haben.

6. Verfahren zur Herstellung von Verbindungen der Formel (I) gemäß Anspruch 1 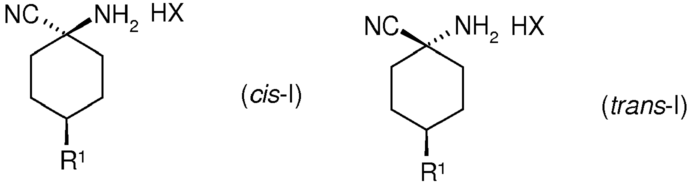 in welcher
R¹ und HX die oben genannten Bedeutungen haben,
**dadurch gekennzeichnet, dass** man Verbindungen der Formel (II) 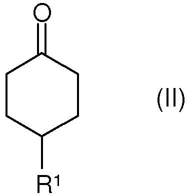 in welcher
R¹ die oben genannten Bedeutung hat,
mit Alkalicyanid, Ammoniumchlorid und Ammoniak in Gemischen von organischen Lösungsmitteln und Wasser im Zwei-Phasen-System zu Verbindungen der Formel (III) 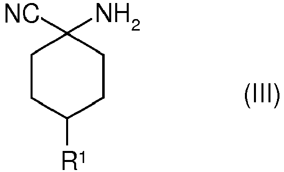 in welcher
R¹ die oben genannten Bedeutungen hat,
umsetzt und diese mit HX versetzt, wobei HX die oben angegebene Bedeutung hat.

7. Verfahren zur Herstellung von Verbindungen der Formel (*cis*-I) gemäß Anspruch 1 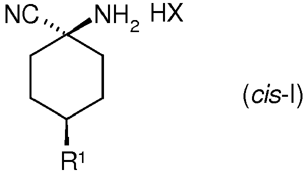 in welcher
R¹ und HX die oben genannten Bedeutungen haben,
**dadurch gekennzeichnet, dass** man Verbindungen der Formel (III) 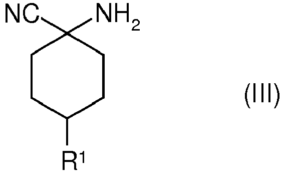 in welcher
R¹ die oben genannten Bedeutungen hat,
in Methyl-tert.-butylether, Toluol, Essigsäureethylester, Essigsäuremethylester oder mit HX umsetzt, wobei HX die oben angegebenen Bedeutungen hat. Tetrahydrofuran und die Abtrennung der Verbindungen der Formel (*cis*-I) durch Filtration erfolgt.

8. Verfahren zur Herstellung von Verbindungen der Formel (III) gemäß Anspruch 6 durch Isomerisierung von Verbindungen der Formel (*trans-*III) und der Formel (*trans*-I) 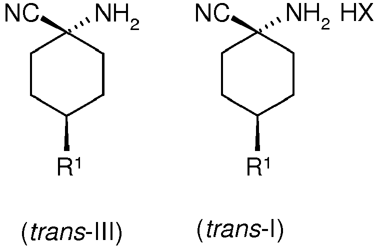 in welchen
R¹ und HX die oben genannten Bedeutung haben,
**dadurch gekennzeichnet, dass** man Verbindungen der Formeln (*trans-*III) und (*trans-*I) mit wässrigem Ammoniak bzw. mit Ammoniak in Gemischen aus organischen Lösungsmitteln und Wasser umsetzt.

9. Verfahren zur Herstellung von Verbindungen der Formel (III) gemäß Anspruch 6 durch Isomerisierung von Verbindungen der Formel (*trans*-III) und der Formel (*trans*-I) 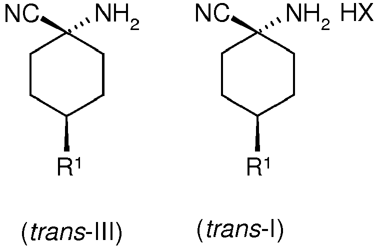 in welchen
R¹ und HX die oben genannten Bedeutung hat,
**dadurch gekennzeichnet, dass** man Verbindungen der Formeln (*trans-*III) und (*trans*-I) zu Verbindungen der Formel (II) beimischt und mit einer zusätzlichen Menge Ammoniak analog Anspruch 8 beschrieben, versetzt und mit Alkalicyanid, Ammoniumchlorid und Ammoniak in Gemischen von organischen Lösungsmitteln und Wasser im Zwei-Phasen-System gemäß Anspruch 6 umsetzt.

## Claims

1. Compounds of the formulae (*cis*-I) and (*trans*-I) 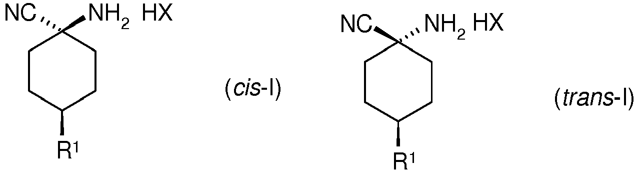 in which
R¹ is OR²,
R² is alkyl,
HX is HCl, H₂SO₄, H₃PO₄, HCOOH, HO₂CCO₂H, p-toluenesulphonic acid or methanesulphonic acid.

2. Compounds of the formulae (*cis*-I) and (*trans*-I) according to Claim 1, in which
R¹ is OR²,
R² is alkyl,
HX is H₂SO₄, H₃PO₄, HCOOH, HO₂CCO₂H, p-toluenesulphonic acid or methanesulphonic acid.

3. Compounds of the formulae (*cis*-I) and (*trans-*I) according to Claim 1, in which
R¹ is OR²,
R² is C₁-C₆-alkyl,
HX is H₂SO₄, H₃PO₄, HCOOH or HO₂CCO₂H.

4. Compounds of the formulae (*cis*-I) and (*trans*-I) according to Claim 1, in which
R¹ is OR²,
R² is methyl, ethyl or n-propyl,
HX is HCOOH.

5. Compounds of the formula (*cis*-I) according to Claim 1, in which R¹ and HX have the meanings given above.

6. Process for the preparation of compounds of the formula (I) according to Claim 1 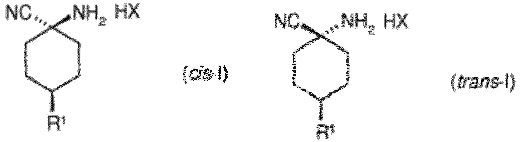 in which
R¹ and HX have the abovementioned meanings,
**characterized in that** compounds of the formula (II) 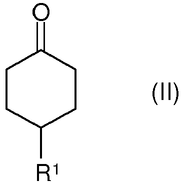 in which
R¹ has the abovementioned meanings,
are reacted with alkali metal cyanide, ammonium chloride and ammonia in mixtures of organic solvents and water in a two-phase system to give compounds of the formula (III) 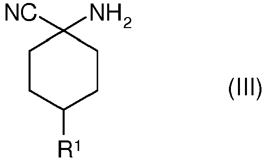 in which
R¹ has the abovementioned meanings,
and these are mixed with HX, HX having the meanings given above.

7. Process for the preparation of compounds of the formula (*cis*-I) according to Claim 1 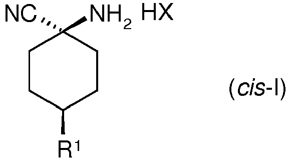 in which
R¹ and HX have the abovementioned meanings,
**characterized in that** compounds of the formula (III) 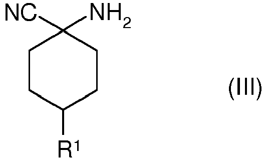 in which
R¹ has the abovementioned meanings,
are reacted with HX, HX having the meanings given above, in methyl tert-butyl ether, toluene, ethyl acetate, methyl acetate or tetrahydrofuran,
and the separation of the compounds of the formula (*cis*-I) is carried out by filtration.

8. Process for the preparation of compounds of the formula (III) according to Claim 6 by isomerisation of compounds of the formula (*trans*-III) and of the formula (*trans*-I) 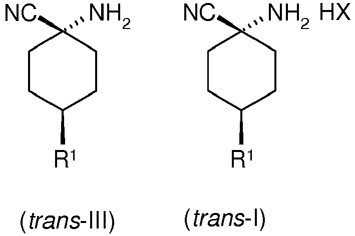 in which
R¹ and HX have the abovementioned meanings,
**characterized in that** compounds of the formulae (*trans*-III) and (*trans*-I) are reacted with aqueous ammonia or with ammonia in mixtures of organic solvents and water.

9. Process for the preparation of compounds of the formula (III) according to Claim 6 by isomerisation of compounds of the formula (*trans*-III) and of the formula (*trans*-I) 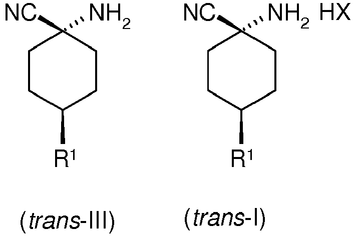 in which
R¹ and HX have the abovementioned meanings,
**characterized in that** compounds of the formulae (*trans*-III) and (*trans-*I) are admixed with compounds of the formula (II) and mixed with an additional amount of ammonia analogously as described in Claim 8 and reacted with alkali metal cyanide, ammonium chloride and ammonia in mixtures of organic solvents and water in a two-phase system according to Claim 6.

## Revendications

1. Composés des formules (*cis*-I) et (*trans*-I) 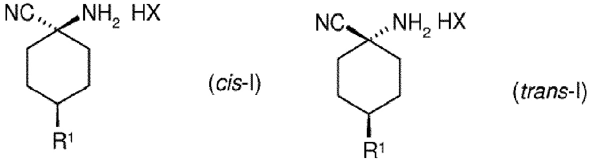 dans lesquelles
R¹ représente OR²,
R² représente alkyle,
HX représente HCl, H₂SO₄, H₃PO₄, HCOOH, HO₂CCO₂H, acide p-toluène-sulfonique ou acide méthane-sulfonique.

2. Composés des formules (*cis-*I) et (*trans*-I) selon la revendication 1, dans lesquelles
R¹ représente OR²,
R² représente alkyle,
HX représente H₂SO₄, H₃PO₄, HCOOH, HO₂CCO₂H, acide p-toluène-sulfonique ou acide méthane-sulfonique.

3. Composés des formules (*cis*-I) et (*trans*-I) selon la revendication 1, dans lesquelles
R¹ représente OR²,
R² représente alkyle en C₁-C₆,
HX représente H₂SO₄, H₃PO₄, HCOOH ou HO₂CCO₂H.

4. Composés des formules (*cis*-I) et (*trans*-I) selon la revendication 1, dans lesquelles
R¹ représente OR²,
R² représente méthyle, éthyle ou n-propyle,
HX représente HCOOH.

5. Composés de formule (*cis*-I) selon la revendication 1, dans laquelle R¹ et HX ont les significations indiquées précédemment.

6. Procédé de fabrication de composés de formule (I) selon la revendication 1 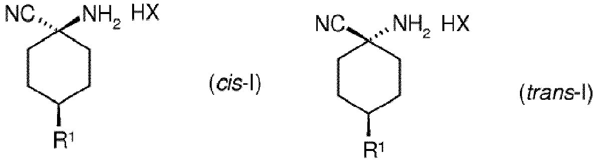 dans lesquelles
R¹ et HX ont les significations indiquées précédemment, **caractérisé en ce que** des composés de formule (II) 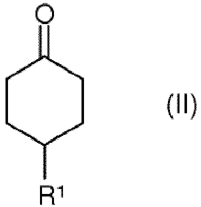 dans laquelle
R¹ a la signification indiquée précédemment,
sont mis en réaction avec un cyanure alcalin, du chlorure d'ammonium et de l'ammoniac dans des mélanges de solvants organiques et d'eau dans un système biphasé pour former des composés de formule (III) 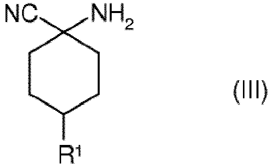 dans laquelle
R¹ a les significations indiquées précédemment,
et ceux-ci sont mélangés avec HX, HX ayant la signification indiquée précédemment.

7. Procédé de fabrication de composés de formule (*cis*-I) selon la revendication 1 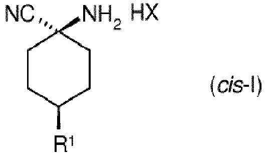 dans laquelle
R¹ et HX ont les significations indiquées précédemment, **caractérisé en ce que** des composés de formule (III) 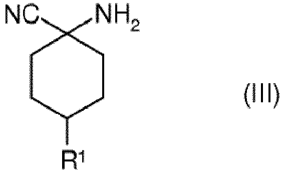 dans laquelle
R¹ a les significations indiquées précédemment,
sont mis en réaction dans de l'éther de méthyle et de tert.-butyle, du toluène, de l'ester éthylique de l'acide acétique, de l'ester méthylique de l'acide acétique ou du tétrahydrofurane avec HX, HX ayant les significations indiquées précédemment,
et la séparation des composés de formule (*cis-*I) a lieu par filtration.

8. Procédé de fabrication de composés de formule (III) selon la revendication 6 par isomérisation de composés de formule (*trans*-III) et de formule (*trans-*I) 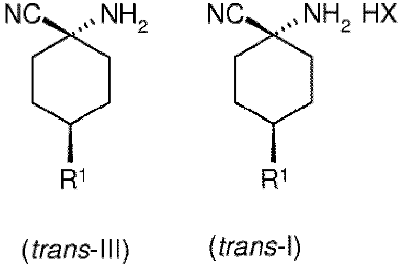 dans lesquelles
R¹ et HX ont la signification indiquée précédemment, **caractérisé en ce que** des composés des formules (*trans-*III) et (*trans*-I) sont mis en réaction avec de l'ammoniac aqueux ou avec de l'ammoniac dans des mélanges de solvants organiques et d'eau.

9. Procédé de fabrication de composés de formule (III) selon la revendication 6 par isomérisation de composés de formule (*trans*-III) et de formule (*trans-*I) 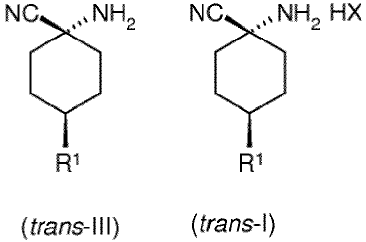 dans lesquelles
R¹ et HX ont la signification indiquée précédemment, **caractérisé en ce que** des composés des formules (*trans-*III) et (*trans*-I) sont ajoutés à des composés de formule (II) et mélangés avec une quantité supplémentaire d'ammoniac tel que décrit dans la revendication 8, et mis en réaction avec un cyanure alcalin, du chlorure d'ammonium et de l'ammoniac dans des mélanges de solvants organiques et d'eau dans un système biphasé selon la revendication 6.
